# EUROPEAN PATENT SPECIFICATION

(11) **EP 2 260 813 B1**
(45) Date of publication and mention of the grant of the patent: **15.07.2015**
(21) Application number: 09425217.8
(22) Date of filing: 29.05.2009
(51) Int. Cl.: B29C 55/04, A61F 13/15, B29C 55/08

(54) **A device and method for forming tensioned elastication strips, for instance for sanitary articles and corresponding computer program product**
Vorrichtung und Verfharen zur Herstellung von gespannte elastizierten Streifen, z.B. für Hygieneartikel und Computerprogrammprodukt für ihre Herstellung
Dispositif et procédé pour la formation de bandes tensionnées rendues elastiques, pour example pour articles hygieniques et program d'ordinateur pour en tel produit

(43) Date of publication of application: 15.12.2010
(73) Proprietor: Fameccanica.Data S.p.A., 66020 Sambuceto di S. Giovanni Teatino (Chieti) (IT)
(72) Inventor: Giuliani, Cristian, 64025 Pineto (Teramo) (IT); Sablone, Gabriele, 65016 Montesilvano (Pescara) (IT); Lombardi, Massimiliano, 65129 Pescara (IT)
(74) Representative: Bosotti, Luciano

(56) References cited:
- EP-A- 0 449 548
- EP-A- 0 672 516
- US-A- 4 925 520
- US-A- 5 407 507
- US-A- 5 545 285

## Description

### Field of the invention

The present disclosure refers to techniques for applying tensioned elastic elements on sanitary articles such as diapers and the like.

The disclosure was devised with particular attention to the possible use for applying tensioned elastic elements along the waistline of sanitary articles wearable like pants. However, reference to this particular application shall not be deemed to be restrictive to the scope of the description.

### Description of the related art

Since the 80s, sanitary articles wearable like pants, such as for example diapers for children, were provided with waist elastic elements, i.e. with elastic material strips cut to size and applied along the waistline of the diaper (on at least one between the front side and the rear side, and preferably on both sides) in order to confer to the diaper better characteristics of adherence to the body of the user, without this leading to an undesired restraint action.

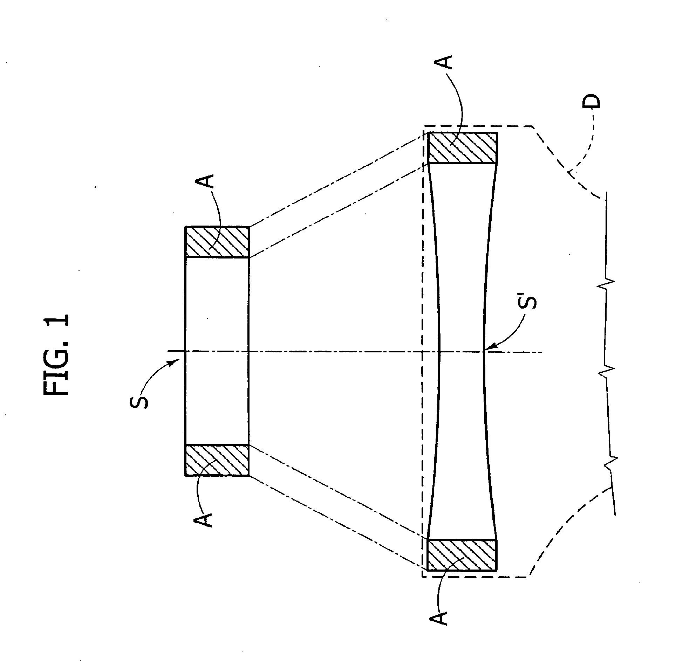

The application of such strips or waist elastic elements implies the performance of the sequence schematically represented in figure 1, i.e.:
- "segmenting" the strips S from a web of elastic material,
- "grasping" (usually at two end grasping regions A) the strip S, in non-stretched condition,
- "pulling" the strip S, stretching it (as schematically indicated with S' in the lower part of figure 1), and
- applying the strip S' maintained stretched on the sanitary article: schematically represented in figure 1 with a dashed line is the profile of one of the ends of a diaper D having, in stretched condition, the typical hourglass configuration.

All this in such a manner that, once released, the strip S is capable of generating the desired elastic return action along the waistline of the article D.

Over the years, the respective technology has undergone various developments, both regarding the material for making the elastic strips (whose characteristics are not entirely independent from the methods of application)and regarding the application techniques(intended to take into account the high production rates typical of the industry).

For example, the first solutions proposed provided for the use, as the waist elastic material, of elastic polyurethane sponge stretchable in all directions. All this with manipulation difficulties linked, for example, to a high permeability such not to allow vacuum gripping thereof.

Still regarding the materials, the use of a material known as "Fluted" (conceived and developed by 3M), made up of a sandwich of two polyethylene sheets with an elastomer interposed therebetween was consolidated in the early 90s. The sheets are coupled together through longitudinal bondings, the joint being obtained using the elastic material in the maximum stretched condition. The material in question is thin, impermeable and elastic only in the transverse direction, with a further advantage given by the small spaces occupied by the material during storage.

The aspects linked to the application technology have concerned both the methods of stretching the material and the method for maintaining the material in the stretched condition.

For example, document JP-A-1 272 803 describes an apparatus in which an elastic material made of a web that is unwound from a reel is initially stretched - after applying glue - in the unwinding direction before being vacuum gripped and segmented into single pieces of tensioned elastic web. The latter are then intended to be rotated by 90° in such a manner to arrange each piece with its larger dimension perpendicular to the direction of movement for the application on a web of sanitary articles undergoing formation.

Alongside this solution (which, due to the treatment of the material width-wise, allows overcoming possible problems linked to the variation of the height of the elastic material itself, with the possibility of processing elastic materials of various types) also employed were other various solutions in which the operation of stretching the elastic material is obtained not operating "length-wise" on an elastic material web, but forming a piece of elastic material that is advanced "cross-wise" (i.e. with its direction of larger extension orthogonal to the advancement direction) by two drawing members that hold the piece against its ends (A, in figure 1). The drawing members have a diverging development and the piece of material to be stretched is "gripped" at its ends by the drawing members in a region where such members are close to each other, i.e. located at a distance approximately equivalent to the length of the piece in a non-stretched condition.

The piece or strip of material to be stretched, arranged bridge-like between the drawing members, is advanced by the members which, following diverging trajectories, determine the stretching of the piece of elastic material. Upon achieving the stretched condition, the material is thus transferred (directly or through one or more return elements, possibly serving as anvil rolls in the cutting operation) onto the final article.

Referring to this general operation criterion are the solutions described in US-A-4 943 340 (where the drawing members are two pairs of opposite diverging trapezoidal belts), US-A-4 925 520, US-A-5 308 345, US-A-5 043 036 or also US-A-5 545 285 where the drawing members are represented by wheels rotating around axes incident or inclined (oblique) with respect to each other, i.e. forming an angle such to determine the degree of divergence of the drawing paths of the ends of the element subjected to stretching and thus the degree of stretching imparted to the element itself.

Thus, such solutions correspond to the common criterion of forming elastication strips applicable in stretched conditions on sanitary articles providing for at least one pair of combined drawing members capable of operating in gripping relation with the ends of the strips to receive the strips arranged bridge-like therebetween in non-stretched condition at an inlet end, where the drawing members are located at a first distance corresponding to the length of the strips in non-stretched condition. The drawing members move following trajectories diverging with respect to each other to draw the strips arranged bridge-like therebetween towards an outlet end, where the drawing members are located at a second distance, greater than the first distance and corresponding to the length of the strips in the elastically stretched condition. The drawing members are provided with gripping formations suitable to interact with the ends of the abovementioned strips.

Such solutions differ from each other due to the methods through which they obtain the gripping on the ends of the strip or elastic element (the ends indicated with A in figure 1).

For example, the solution of US-A-4 925 520 provides for a vacuum gripping assisted by elements that penetrate into the elastic material on the edges, while the solutions of US-A-5 308 345 and US-A-5 043 036 provide for that the gripping ends of the single stretched element be pinched in a peripheral groove formed in the disc or wheel representing the drawing member and a belt that is wound in such throat. A somewhat similar arrangement is disclosed, in connection with stretching a moving elastomeric web, in EP-A-0 672 516 or EP-A-0 449 548. In the case of US-A-5 545 285, the retention action of the ends of the strip subjected to stretching is performed by mechanical "hands" controlled through a cam system.

More specifically, the invention relates to a device according to the preamble of claim 1, which is known, e.g. from US-A-5 407 507.

### Object and summary of the invention

The inventors observed that the solutions described in the abovementioned documents leave the problem of performing the "change of format" operation in a simple and reliable manner unsolved.

In the context considered herein, the expression "change of format" generally indicates the operation or the entirety of the operations intended to allow that an apparatus for applying waist elastic elements for sanitary articles passes from the obtainment of an article of given dimensions to the obtainment of an article of different dimensions.

Regarding this case, the change of format essentially concerns the "pitch" for applying waist elastic elements on a chain of sanitary articles wearable like pants during formation. Passing from a "small" article (for example a diaper for newborns) to a "large" article (for example a diaper for one or two year old children) requires increasing the pitch for applying elastic strips S'. On the contrary, passing from a large article to a small article requires reducing the application pitch.

It shall be observed that this problem arises generally, regardless of the specific methods of obtaining the single article, and in particular regardless of the method of applying two waist elastic elements to the two ends of each article.

For example, application of stretched elastic elements with a pitch corresponding to the length of the single article is provided for in some solutions. Each elastic element is then intended to be cut into two in the longitudinal direction when the chain of articles being formed is divided into single articles, this allowing that each elastic element be divided into two parts: one is associated to the rear/front edge of an article and the other is associated to the front/rear edge of the successive article in the catena.

In other solutions, two successive operations of applying waist elastication elements may be provided for: the first for applying, with a pitch corresponding to the length of the single articles, the elastication elements of the rear edges and the second for applying, still with a separation pitch equivalent to the length of the single articles, the elastication elements of the rear edges of the articles.

Per se, the specific methods have no impact on the problem regarding the change of format in the general terms through which the same is tackled herein.

Specifically, the inventors observed that, in some of the solutions described beforehand, the operation of changing the format requires replacing part of the apparatus used (for example the end parts of the diverging wheels) or performing considerable readjustment operations. Still according to the observations of the inventors, in an attempt to overcome the problem regarding the change of format it is also important to avoid problems linked to the mechanism of gripping and retaining the strips S, S' subjected to stretching. In particular, it is important to prevent the mechanism of drawing and stretching the strips S from being contaminated by the adhesive material intended to retain the strips S' once stretched in the waist application position on the sanitary articles. The inventors also noticed that the lubricants or release agents useable to prevent or reduce such contamination phenomena may have negative effects on the action of retaining the elastic elements by the diverging device used for stretching the strips, with possible slipping capable of turning into a oblique application of the elasticising elements.

Thus, the present invention has the object of providing a solution capable of, on one hand, overcoming the problem of change of format and, on the other, overcoming the previously outlined drawbacks. According to the present invention, such object is attained due to a device having the characteristics specifically referred to in the claims that follow. The invention also regards a corresponding method, as well as a computer program product, loadable into the memory of at least one computer and including software code portions suitable to implement the steps of the method when the product is executed on at least one computer. As used herein, reference to such computer program product is intended to be equivalent to the reference to a computer readable medium containing instructions for the control of a processing system to coordinate the implementation of the method according to the invention. Reference to "at least one computer" is obviously intended to highlight the possibility that the present invention be implemented in modular and/or distributed form. The claims form an integral part of the technical disclosure provided herein in relation to the invention.

### Brief description of the attached representations

Now, the invention shall be described, strictly for exemplifying and non-limiting purposes, with reference to the attached representations, wherein:
- figure 1 has already been described previously,
- figure 2 illustrates one of the devices included in the apparatus described herein,
- figure 3 is a view, reproduced in an enlarged scale, of the part of figure 2 corresponding to arrow III, and
- figure 4 is an elevational schematic view of an apparatus as described herein.

### Detailed description of embodiments

Illustrated in the following description are various specific details aimed at an in-depth understanding of the embodiments. The embodiments may be obtained without one or more specific details, or through other methods, components, materials etc. In other cases, known structures, materials or operations are not shown or described in detail to avoid obscuring the various aspects of the embodiments.

Reference to "an embodiment" in this description indicates that a particular configuration, structure or characteristic described regarding the embodiment is included in at least one embodiment. Hence, expressions such as "in an embodiment", possibly present in various parts of this description do not necessarily refer to the same embodiment. Furthermore, particular configurations, structures or characteristics may be combined in any suitable manner in one or more embodiments.

References herein are used for facilitating the reader and thus they do not define the scope of protection or the scope of the embodiments.

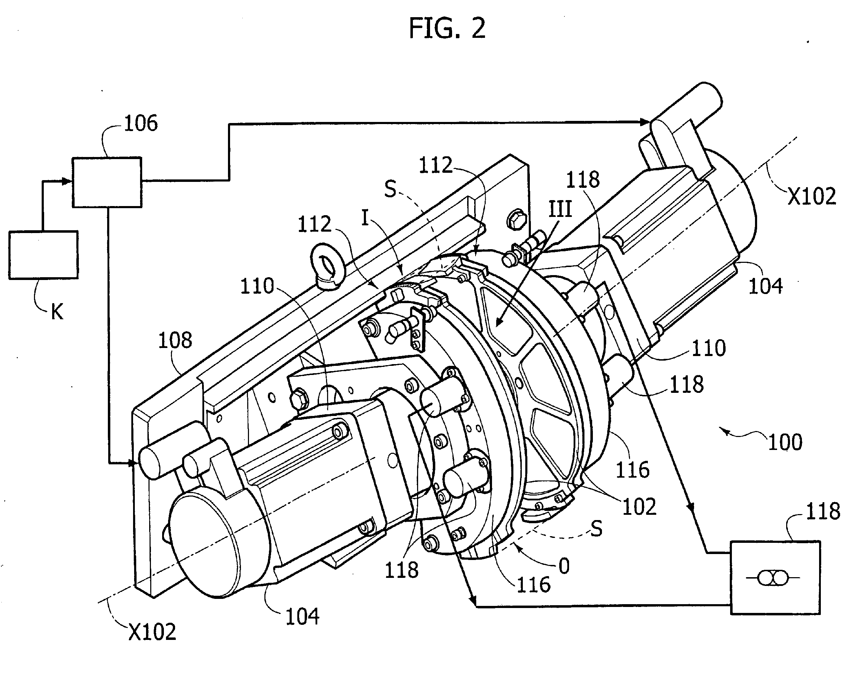

In figures 2 - 4, elements, parts and components identical or equivalent to elements, parts and components described previously were indicated using the same reference numbers and shall not be described further herein for the sake of brevity.

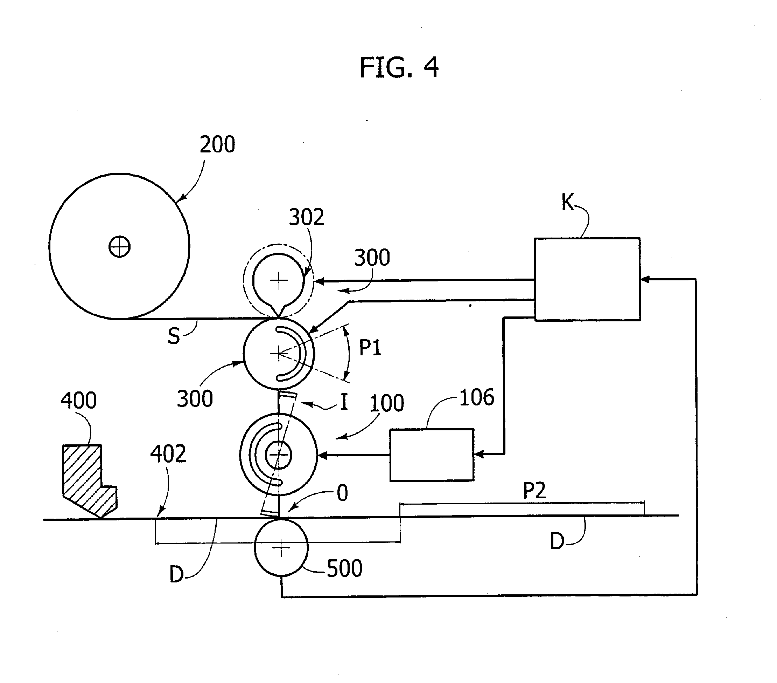

Figure 2 illustrates a general perspective view of a device 100 included in an apparatus for applying elasticised elements (strips) on the waistline of sanitary articles such as diapers D. Tale Such apparatus shall be described in a more complete manner with reference to figure 4.

The part of apparatus indicated with 100 (hereinafter referred to as "diverging device" for the sake of brevity) includes two motorised wheels or discs 102, for example with spoke structure, arranged in position facing the respective rotation axes X102 intersected with respect to each other in an intermediate point between the two wheels or discs 102.

Mounted on the wheels 102, in a peripheral position, are gripping formations 112 (so-called "shoes") intended to grip the ends A of the strips thus serving as a member for drawing the strips subjected to stretching.

Each of these gripping formations or shoes 112 (each of the two wheels 102 has at least one or at least one pair of two formations 112 arranged in a position diametrically opposite) is intended to cooperate with a homologous formation 112 arranged in a corresponding position on the other wheel 102 in such a manner to obtain a gripping and traction or longitudinal stretching action on the two ends A of a respective strip S arranged bridge-like between the peripheral of the two wheels 102.

The diverging device 100 is supported by a plate 108 in such conditions wherein, in the trajectories travelled by the peripheries of the wheels 102, thus by the formations 112, an inlet portion I and an outlet portion or are generally distinguishable. The terms "inlet" and "outlet" refer to the movement trajectory of the strips S, S' drawn through the apparatus itself by the formations 112.

The mutually inclined (oblique) or angled arrangement of the axes X102 of the wheels 102 causes the peripheries of the wheels 102, and thus the trajectories travelled by the formations 112 serving as drawing members for the strips subjected to stretching, to lie in planes forming an ideal dihedron therebetween, in such a manner to be "close" (i.e. located at a first distance) at the inlet end I and "apart" (i.e. located at a second distance, greater than the first) at the outlet end O.

In particular:
- when located in the inlet zone I, where the peripheries of the wheels 102 are "close" to each other, the homologous formations 112 arranged in corresponding positions on the two wheels 102 are at a first distance corresponding to the length of the strips S before being stretched, and
- when located in the outlet zone O, where the peripheries of the wheels 102 are "apart" from each other, the homologous formations 112 arranged in corresponding positions on the two wheels 102 are at a second distance, about equivalent and in any case corresponding to the length of the strips S' in stretched condition, and thus greater than the abovementioned first distance.

A strip element S applied bridge-like between homologous formations 112 on the two wheels 102 at the inlet end I and then drawn by the wheels 102 in the orbital movement, shall thus be in stretched condition S' upon reaching the outlet end O. At the outlet end O, the strip S' (in elastically stretched condition) may be applied - directly or indirectly - on the waistline of a sanitary article D in such a manner to attain the elastication regarding the same.

This general operation principle is per se known in the art (in particular it is discussed in various documents mentioned in the introduction of the present description) and thus does not require a further detailed description herein.

The degree of divergence of the axes X102, thus the degree of divergence of the dihedron plane in which the trajectories travelled by the formations 112 lie, may be made adjustable (for example by providing for mounting wheels 102 on brackets 110 whose fixing position on the plate 108 is selectively adjustable) in such a manner to vary the ratio between the distances that separate the formations 112 at the inlet end I and at the outlet end O, thus varying the degree of stretching of the strips.

In an embodiment, the two wheels 102 are drawn in rotation (clockwise, with respect to the point of observation of figure 2) by respective motors 104 driven by a control module 106 in turn operated under the control of a control device K (such as a so-called PLC or un Personal Computer for industrial use) which supervises the general operation of the apparatus in which the diverging device 100 is inserted.

The motors 104 are synchronised with respect to each other in such a manner to prevent the single strip S deposited bridge-like between the two formations 112 from ending up oblique due to a rotation speed difference of the wheels 102. Otherwise, as better explained hereinafter, such rotation speed (common to the two wheels 102) is selectively controllable in order to obtain the "change of format" according to the conditions described hereinafter.

In the illustrated embodiment, the gripping action exerted by the formations 112 on the ends A of the strips is of two types: by vacuum and by mechanical hooking or interference.

Firstly, there is a vacuum gripping action obtained through a series of openings 114 provided in the formations 112 and controlled, through a collector 116 and connection mouths 118 (all elements being fixed and thus not following the formations 112 in the orbital movement imparted thereon due to the rotation of the wheels 102) by a source of subatmospheric pressure (e.g. a so-called "vacuum pump") 118 intended to introduce an airflow from the environment into the collectors 116 through the openings 114.

Being located above such openings 114, each end A of a strip closes such openings and thus it is forced (and actually "seized") with a typical vacuum gripping effect on the gripping formation 112.

By adjusting the intensity of the action of the pump 118 it is possible to selectively adjust the intensity of the gripping action exerted through the openings 114 on the end A of the strips. According to per se known criteria, it is possible to obtain the collectors 116 in such a manner that the abovementioned vacuum gripping action by the formations 112 starts to be exerted, for example, at the inlet end I of the diverging device 100 before ceasing - in any case - at the outlet end O.

All this in such a manner to ensure that the strips are:
- taken over by the formations 112 in non-stretched conditions (S) at the inlet end I,
- drawn by the formations 112 in their orbital movement towards the outlet end or and thus brought to the stretched condition (S') through the stretching mechanism referred to previously, and
- released by the formations 112 so as to be transferred onto the articles D, where the strips S' in stretched condition are fixed (for example through adhesive means) onto the articles D themselves.

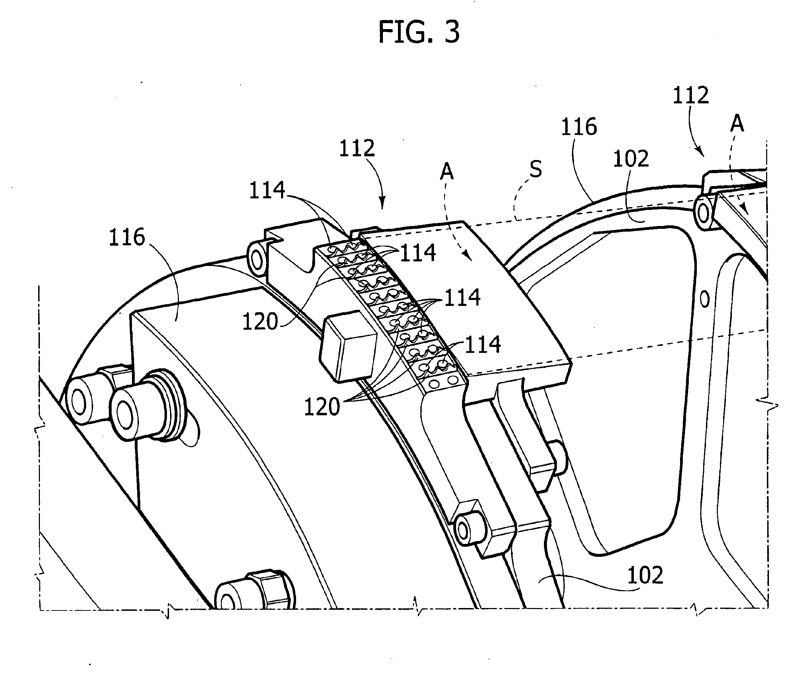

As better observable in the views of figure 3, the retention action of the ends A of the strips S, S' is made firmer by the presence, for example along the contour of the holes 114, of hooking formations 120 substantially similar to, in the embodiment illustrated herein, pieces of sawblade, whose teeth preferably have the steeper edge facing outwards the wheels 102.

The "vacuum" which operates through the openings 114 presses the ends A against the surfaces of the gripping and retention formations 112. The teeth 120 (or analogous hooking formations), though not necessarily penetrating into the end A, "seize" it obtaining, in synergy with the vacuum gripping effect, a retention action which:
- is extremely firm: the ends A, when simultaneously restrained by the vacuum, and the teeth 120, move neither in axial nor perimeter direction with respect to the formations 112 though the wheels 102 that bear them are subjected - as better observed hereinafter - to even quite sudden speed variations; and
- when the retention action of the vacuum exerted through the openings 114 ceases, it allows the strips S' in stretched condition to be easily separated from the formations 112, without hindrances and without any considerable drawing effect by the formations 112 themselves.

Figure 4 illustrates the general structure of the apparatus or system in which the diverging device 100 is included.

In the diagram of figure 4, reference 200 indicates a reel or an analogous source of supply from which material made of elastically stretchable web such as, for example, CEX-816 material produced by Tredegar from Richmond, Virginia (USA) is unwound. Such web is subjected to segmentation (i.e. cutting in transverse direction with respect to the direction of unwinding from the reel 200) and generates single strips S.

The material that is unwound from the reel 200 (indicated briefly with S to indicate its purpose to form the strips S) is subjected to the abovementioned segmentation action in a cutting unit 300 of the rotating knife type, for example. Such unit is formed by a knife 302 and by an anvil roll 304, both rotating (respectively anticlockwise for the knife 302 and clockwise for the anvil roll 304, in the example illustrated herein) depending on the controls coming from the control unit K.

In the representation of figure 4, which is obviously a schematic representation, the fact that two control lines start from the unit K towards the knife 302 and the anvil roll 304 shows that the abovementioned elements of the cutting unit 300 are not necessarily required to rotate at the same tangential velocity with respect to each other. In particular, the cutting unit 300 may be of the type currently referred to as "cut & slip", in which the material S subjected to cutting is capable of slipping on the surface of the anvil roll 304. This allows enabling the single strips S (cut-out from the web 200 with a pitch P1 actually corresponding to the "height" of the single strip S) to be spaced and be supplied from the anvil roll 304 to the diverging device 100 already spaced from each other, facilitating the gripping action by the diverging device 100 itself.

Regardless of the solution adopted for the cutting unit 300 (with or without slipping), the single strips S are taken over by the diverging device 100 (arranged bridge-like between the two wheels 102, with the two ends A restrained by a pair of gripping formations 112, one on each of the wheels 102) with a velocity (tangential, of the contour of the wheels 102) V1.

In an embodiment, the velocity V1 corresponds to the pitch P1 according to the relation V1=P1 x n, where n indicates the number article per time unit D (see the lower part of figure 4) on which the strips (once obtained the stretched condition S') are intended to be applied with a pitch P2.

In figure 4 reference 400 indicates an application device (per se known) which periodically applies - with pitch P2 - on the chain of the articles D still connected to each other, adhesive areolae 402 on which the strips S' in stretched conditions are applied.

Reference number 500 generally indicates a roll intended to support a chain of articles D at the point of application of the strips S' starting from the diverging device 100.

In the embodiment illustrated herein, the roll 500 is also identified as a source of synchronism which, identifying the advancement velocity of the articles D, delivers to the control unit K a general synchronism signal that allows adjusting, according to criteria better described hereinafter, the operation velocity of the various devices included in the apparatus considered herein. As known to those skilled in the art, instead of a moveable element (rotating, for example such as the roll 500) serving as a source of "master" synchronism signal, such synchronism may also derive from a clock generator serving as a virtual master.

In the embodiment illustrated herein, the rotation velocity of the wheels 102 (thus the velocity of the orbital movement of the formations 112 serving as members for drawing the strips through the diverging device 100) is not constant, but varies following a predetermined profile, set by the control unit K through the control module 106 of the motors 104.

All this in such a manner to ensure that the strips S' (it should be borne in mind that such reference is used to indicate the strips in stretched condition) are applied on the articles D with a velocity V2=P2 x n where n indicates, once again, the number of articles D obtained within a time unit, while P2 is the application pitch of the elements of the elasticised strips S' on the articles D.

The pitch P2 may vary according to the article obtained, and the variation or adjustment of the pitch P2 expresses the "change of format" operation.

Regarding reference to the articles D, it shall be observed that, when applying the elasticised strips S', the articles D are usually still incomplete: the application of the strips S' represents one of the operations for obtaining such articles. For example, when applying the elasticised strips S', the articles D may also comprise only the sheet of the backsheet (respectively, topsheet), on which the absorbent core and the topsheet (respectively, backsheet) are subsequently intended to be applied, with the possible interposition or addition of all components (additional absorbent layers such as acquisition layers or the like, side containment cuffs, etc...) suitable to be included in such articles D.

The motorisation solution used for the wheels 102 - motors 104 susceptible to be controlled according to a selectively determinable rotation velocity profile, also in terms of acceleration and jerk - allows selecting the velocity value V2 (and thus the pitch value P2, in order to achieve a "change of format") in an entirely independent manner, for example, from the velocity at which the strips themselves are moved to the inlet of the diverging device 100.

All this being obtained by intervening in a simple manner (for example at software level) on the unit K and/or on the module 116, without requiring replacing the parts and/or performing complex adjustment operations. In particular, the respective information technology product, loadable into the unit K and/or into the module 106, may acquire the characteristic of a retrofit product of an already existent apparatus.

In cases where the cutting device 300 does not provide for the slipping of the material S subjected to cutting, the strips S may be supplied to the diverging device 100 (wherein, for the sake of simplicity, each wheel 102 is considered provided with only one gripping formation 112) with a velocity V1=P1 x n, where P1 is the cutting pitch of the device 300, i.e. the "height" of each strip S.

The rotation of the wheels 102 is then controlled (by the control device K and by the module 106, through the motors 104) in such a manner that, when passing from the inlet end I of the diverging device 100 to the outlet end O, when the stretched strips S' are transferred on the articles D, the tangential velocity of the formations 112 (which maintain their position facing the two wheels 102) reaches, due to an acceleration, a value V2=P2 x n, where P2 is the application pitch desired on the articles D.

The "change of format" operation may in this case be obtained by simply modifying (for example through software) the operation for controlling the motors 104 by the unit K and the module 106 imparting to the wheels 102, while the gripping formations 112 pass from the inlet end I to the outlet end O, an acceleration ramp such to transfer the tangential velocity of the formations 112 from value V1 to value V2, according to the desired pitch P2 (hence according to the desired format). All this with an extremely wide range of choice.

During the return of the gripping formations 112 (which have just released a strip S' applying it on the article D at the outlet end O) towards the inlet end I of the diverging device 100, the tangential velocity of the formations 112 is once again slowed down (by operating on the motorisation of the wheels 102) in such a manner to return it to value V1.

Should the cutting unit 300 be of the type that allows the slipping (for example a "cut & slip" unit of the "pitchless" type, as described, for example, in EP-A-1 864 768) it can be provided for that, though being cut with a pitch P1, the strips S be drawn by the anvil roll 304 already at velocity V2 corresponding to the pitch P2. This, due to the fact that the velocity of the anvil roll 304 is controllable by the unit K independently from that of the knife 302.

In this case, the transfer of the single strip S from the anvil roll 304 to the formations 112 of the diverging device 100 may already occur at velocity V2.

In this case, as schematically illustrated in figure 4, the wheels 102 may each be provided with two formations 112 diametrically opposite from each other. This, in such a manner that, while a pair of such formations 112 receives a strip S to be stretched from the anvil roll 304 (at velocity V2), a pair of formations 112 in a diametrically opposite position is capable of transferring a strip in stretched condition S' on the chain of articles D. In such conditions, both gripping formations 112 present on each wheel 102 actually serve their purpose at a tangential velocity equivalent to V2.

Furthermore, it shall be observed that that the hypothetical maintenance of the rotation velocity V2 = P2 x n constant for the wheels 102 does not lead to applying subsequent stretched strips S' with the desired pitch P2. As a matter of fact, such condition could only be met if P2=Π x R i.e. ½ Π x D, where R and D indicate the radius and the diameter, respectively, of the orbital trajectory of the gripping formations 112. Such condition, inherently linked to the geometry of the diverging device 100 (in practice to the dimensions of the wheels 102) is actually met only at a pitch value, which overtly contradicts the object of performing the change of format operation in a simple and most automatic manner possible.

Hence, also in this embodiment, the control unit K intervenes, through the module 106, on the motorisation 104 of the wheels 102 modulating the velocity of the wheels 102 in the intermediate regions between the two angular positions in which they are obtained, twice at each rotation of the wheels:
- on one side, the gripping of a strip S to be stretched starting from the anvil roll 304, and
- on the opposite side, the application on the chain of articles D of a stretched strip S'
   in such a manner that the mean velocity Vd of the orbital movement of the formations 112 borne by the wheels 102 corresponds to the relation Vd = Π x R = 1/2 Π x D such to ensure that the (pairs of) formations 112 intended to apply the stretched strips S' on the flow of articles D correctly reach the contact with the region of each article D where the adhesive formations 402 are found applied in the station 400 or, generally, the region of the single article D where the stretched strip S' is applied.

It shall be observed that the methods for retaining and releasing the strips S by the formations 112 combine with the operation methods described previously in that the variation of the rotation velocity of the wheels 102 is not conditioned, and does not condition, the gripping and release mechanism of the strips S, S' as - in the contrary - it would occur with different gripping solutions such as solutions based on the use of belts or mechanical "hands" which, exerting a fastening (or "pinching") action on the strips S would inevitably create an obstruction on the outer surface of the strips S preventing quick and immediate release thereof on the articles D precisely at the desired instant. At the same time, the described gripping and retention methods are capable of ensuring that the strips S follow the formations 112 even in presence of quick angular velocity variations of the wheels 102 (which can be accompanied by acceleration and jerk values even of considerable level).

Obviously, without prejudice to the principle of the invention, the details and embodiments may vary, even significantly, with respect to what has been described herein by way of non-limiting example only, without departing from the scope of the invention as defined by the attached claims.

## Claims

1. Device for forming elastication strips for application in stretched condition (S') on sanitary articles (D), including at least one pair of combined drawing members (112) to operate in gripping relation on the ends (A) of said strips to receive said strips arranged bridge-like therebetween in non-stretched condition (S) at an inlet end (I) of the device (100), where said drawing members (112) are at a first distance corresponding to the length of said strips (S) in non-stretched condition (S); said drawing members (112) being moveable (102, 104) following trajectories diverging from each other to draw said strips arranged bridge-like therebetween towards an outlet end (0) of the device (100), where said drawing members are at a second distance, greater than said first distance and corresponding to the length of said strips in elastically stretched condition (S), the device including a supply unit (300) for supplying said strips in non-stretched condition (S) to said inlet end (I) of the device (100) with a given supply pitch (P1),
**characterised in that** said drawing members (112) are moved by motor means (104) with selectively variable velocity (106, K) when transferring said strips between said inlet end (I) and said outlet end (O) to selectively vary the application pitch (P2) of said strips in stretched condition (S') on said sanitary articles (D), the variation of the speed of said drawing members (112) between said inlet end (I) and said outlet end (O) determining the application pitch (P2) of said strips (S') in stretched condition on said sanitary articles (D).

2. Device according to claim 1, wherein said drawing members (112) operate on the ends of said strips (A) in a gripping relation without fastening or pinching.

3. Device according to either of claims 1 or 2, wherein:
- a supply unit (300) is provided for supplying said strips (S) in non-stretched condition to said inlet end (I) of the device (100) at a velocity corresponding to the application velocity (V2) of said strips in stretched condition (S') on said sanitary articles (D), and
- each of said drawing members (112) is included in a pair of opposite drawing members (112) hence, when one drawing member (112) of the pair is located at said inlet end (I), the other drawing member (112) of the pair is located at said outlet end (O).

4. Device according to any one of the preceding claims, wherein said drawing members (112) are borne by wheels (102) arranged in position facing each other with the respective rotation axes (X102) incident and oblique with respect to each other, the inclination of said axes (X102) determining the ratio between said second distance and said first distance and the ensuing degree of stretching of said strips in said stretched condition (S').

5. Device according to claim 3 and 4, wherein said pair of opposite drawing members (112) includes drawing members (112) borne in diametrically opposite positions by one of said wheels (102) arranged in position facing each other.

6. Device according to claim 1 or 3, wherein said supply unit (300) is a cutting device (302, 304) which produces said strips (S) starting from a material web (200).

7. Device according to any one of claims 4 or 5, in combination with claim 6, wherein said cutting device is a cutting device of the cut and slip type, suitable to space said strips (S) from each other when the same are cut starting from said web (200).

8. Device according to any one of the preceding claims wherein said drawing members (112) include, at least one, and preferably both of:
- a vacuum gripping unit (114), and
- hooking formations (120),
to act on the ends (A) of said strips (S, S').

9. Device according to claim 8, wherein said vacuum gripping unit (114), if provided for, is couplable to a subatmospheric pressure source (118) through a pneumatic connection line (116, 118) wherein the application of subatmospheric pressure to said vacuum gripping unit (114) is selectively deactivatable at said outlet end (0).

10. Device according to any one of claims 8 or 9, wherein said hooking formations (120) have a general sawtooth profile, preferably with the steep side of the sawtooth facing outwards the pair of combined drawing members (112).

11. Device according to any one of claims 8 to 10, wherein said hooking formations are arranged in interposed position between said vacuum gripping elements (114).

12. Method for forming elastication strips for application in stretched condition (S') on sanitary articles (D) using at least one pair of combined drawing members (112) operating in gripping relation on the ends (A) of said strips, the method including:
- supplying said strips in non-stretched condition (S) to said drawing members (112) with a given supply pitch (P1),
- receiving said strips arranged bridge-like in non-stretched condition (S) between said drawing members (112) located at a first distance corresponding to the length of said strips (S) in non-stretched condition (S);
- moving said drawing members (112) following trajectories diverging from each other, thus said drawing members draw said strips arranged bridge-like therebetween with said drawing members (112) located at a second distance, greater than said first distance and corresponding to the length of said strips in elastically stretched condition (S),
**characterised in that** it includes moving said drawing members (112) with selectively variable velocity (106, K) when transferring said strips to selectively vary the application pitch (P2) of said strips in stretched condition (S') on said sanitary articles (D), the variation of the speed of said drawing members (112) determining the application pitch (P2) of said strips (S') in stretched condition on said sanitary articles (D).

13. Method according to claim 12, including operating said drawing members (112) on the ends of said strips (A) in a gripping relation without fastening or pinching.

14. Method according to claim 12 or 13, including applying said elastication strips in stretched condition (S') on said sanitary articles (D).

15. Method according to any one of claims 12 to 14, actuated with the device according to any one of claims 1 to 11.

16. Computer program product loadable in the memory of at least one computer (K, 106) and including software code portions configured to actuate the operation of moving said drawing members (112) with selectively variable velocity (106, K) when transferring said strips to selectively vary the application pitch (P2) of said strips in stretched condition (S') on said sanitary articles (D) in the method according to any one of claims 12 to 15 when the product is run on a computer.

## Patentansprüche

1. Vorrichtung zur Bildung von Elastifizierungsstreifen zum Aufbringen im gedehnten Zustand (S') auf Hygieneartikeln (D), umfassend mindestens ein Paar von kombinierten Ziehelementen (112), die in Greifbeziehung auf den Enden (A) der Streifen wirksam werden, um die dazwischen stegartig angeordneten Streifen im ungedehnten Zustand (S) an einem Einlassende (I) der Vorrichtung (100) aufzunehmen, wobei sich die Ziehelemente (112) in einem ersten Abstand befinden, der der Länge der Streifen (S) im ungedehnten Zustand (S) entspricht; die Ziehelemente (112) bewegbar sind (102, 104) nach Bahnkurven, die von einander abzweigen, um die dazwischen stegartig angeordneten Streifen zu einem Auslassende (O) der Vorrichtung (100) hin zu ziehen, wobei sich die Ziehelemente in einem zweiten Abstand befinden, der größer ist als der erste Abstand und der Länge der Streifen im elastisch gedehnten Zustand (S) entspricht, wobei die Vorrichtung eine Zuführeinheit (300) enthält, um die Streifen im ungedehnten Zustand (S) dem Einlassende (I) der Vorrichtung (100) mit einer vorgegebenen Zuführstufe (P1) zuzuführen;
**dadurch gekennzeichnet, dass** die Ziehelemente (112) durch Antriebsmittel (104) mit selektiv veränderlicher Geschwindigkeit (106, K) bewegt werden, wenn die Streifen zwischen dem Einlassende (I) und dem Auslassende (O) verlegt werden, um den Aufbringabstand (P2) der Streifen im gedehnten Zustand (S') auf die Hygieneartikel (D) selektiv zu verändern, wobei die Änderung der Geschwindigkeit der Ziehelemente (112) zwischen dem Einlassende (I) und dem Auslassende (O) den Aufbringabstand (P2) der Streifen (S') im gedehnten Zustand auf die Hygieneartikel (D) bestimmt.

2. Vorrichtung nach Anspruch 1, wobei die Ziehelemente (112) auf den Enden der Streifen (A) in einer Greifbeziehung arbeiten, ohne sich festmachen zu lassen oder einzuklemmen.

3. Vorrichtung nach einem der Ansprüche 1 oder 2, wobei:
- eine Zuführeinheit (300) zum Zuführen der Streifen (S) im ungedehnten Zustand an das Einlassende (I) der Vorrichtung (100) mit einer Geschwindigkeit, die der Aufbringgeschwindigkeit (V2) der Streifen im gedehnten Zustand (S') auf die Hygieneartikel (D) entspricht, vorgesehen ist, und
- jedes der Ziehelemente (112) in ein Paar gegenüber liegender Ziehelemente (112) einbezogen ist, folglich sich das andere Ziehelement (112) des Paars an dem Auslassende (O) befindet, wenn sich ein Ziehelement (112) des Paars an dem Einlassende (I) befindet.

4. Vorrichtung nach einem der vorhergehenden Ansprüche, wobei die Ziehelemente (112) von Rädern (102) getragen werden, die einander gegenüber liegend in der richtigen Lage mit den jeweiligen Rotationsachsen (X102) angeordnet sind, einfallend und schräg relativ zueinander, wobei die Schrägstellung der Achsen (X102) das Verhältnis zwischen dem zweiten Abstand und dem ersten Abstand und den nachfolgenden Dehnungsgrad der Streifen in dem gedehnten Zustand (S') bestimmt.

5. Vorrichtung nach Anspruch 3 und 4, wobei das Paar gegenüber liegender Ziehelemente (112) Ziehelemente (112) einschließt, die in diametral entgegen gesetzten Positionen von einem der Räder (102) getragen werden, die einander gegenüber liegend in der richtigen Lage angeordnet sind.

6. Vorrichtung nach Anspruch 1 oder 3, wobei die Zuführeinheit (300) eine Schneidvorrichtung (302, 304) ist, welche die Streifen (S) ausgehend von einer Materialbahn (200) erzeugt.

7. Vorrichtung nach einem der Ansprüche 4 oder 5, in Verbindung mit Anspruch 6, wobei die Schneidvorrichtung eine Schneidvorrichtung des Schneid- und Gleittyps ist, geeignet um die Streifen (S) im Abstand voneinander anzuordnen, wenn dieselben von der Bahn (200) ausgehend geschnitten werden.

8. Vorrichtung nach einem der vorhergehenden Ansprüche, wobei die Ziehelemente (112):
- eine Unterdruckgreifeinheit (114) und/oder
- Einhakprofilierungen (120),
die auf den Enden (A) der Streifen (S, S') wirksam werden, oder beides umfassen.

9. Vorrichtung nach Anspruch 8, wobei die Unterdruckgreifeinheit (114), falls dafür vorgesehen, durch eine pneumatische Verbindungsleitung (116, 118) an eine subatmosphärische Druckquelle (118) anschließbar ist, wobei die Anwendung von subatmosphärischem Druck auf die Unterdruckgreifeinheit (114) an dem Auslassende (O) selektiv außer Betrieb setzbar ist.

10. Vorrichtung nach einem der Ansprüche 8 oder 9, wobei die Einhak¬profilierun¬gen (120) ein allgemein übliches Sägezahnprofil aufweisen, wobei die steile Seite des Sägezahns vorzugsweise von dem Paar kombinierter Ziehelemente (112) nach außen weist.

11. Vorrichtung nach einem der Ansprüche 8 bis 10, wobei die Einhakprofilierungen in dazwischen gelegter Position zwischen den Unterdruckgreifelementen (114) angeordnet sind.

12. Verfahren zur Bildung von Elastifizierungsstreifen zum Aufbringen im gedehnten Zustand (S') auf Hygieneartikeln (D) mittels mindestens einem Paar von kombinierten Ziehelementen (112), die in Greifbeziehung auf den Enden (A) der Streifen wirksam werden, wobei das Verfahren umfasst:
- Zuführen der Streifen in ungedehntem Zustand (S) an die Ziehelemente (112) mit einem vorgegebenen Zuführgrad (P1),
- Aufnehmen der stegartig angeordneten Streifen in ungedehntem Zustand (S) zwischen den Ziehelementen (112), die in einem ersten Abstand angeordnet sind, der der Länge der Streifen (S) in ungedehntem Zustand entspricht;
- Bewegen der Ziehelemente (112) nach Bahnkurven, die voneinander abzweigen, so die Ziehelemente die dazwischen liegenden, stegartig angeordneten Streifen ziehen, wobei die Ziehelemente (112) in einem zweiten Abstand angeordnet sind, der größer ist als der erste Abstand und der Länge der Streifen im elastisch gedehnten Zustand (S) entspricht, **dadurch gekennzeichnet, dass** es das Bewegen der Ziehelemente (112) mit selektiv veränderlicher Geschwindigkeit (106, K) umfasst, wenn die Streifen verlegt werden, um den Aufbringabstand (P2) der Streifen in gedehntem Zustand (S') auf den Hygieneartikeln (D) selektiv zu verändern, wobei die Änderung der Geschwindigkeit der Ziehelemente (112) den Aufbringabstand (P2) der Streifen (S') in gedehntem Zustand auf den Hygieneartikeln (D) bestimmt.

13. Verfahren nach Anspruch 12, umfassend das Handhaben der Ziehelemente (112) an den Enden der Streifen (A) in einer Greifbeziehung, ohne sich festmachen zu lassen oder einzuklemmen.

14. Verfahren nach Anspruch 12 oder 13, umfassend das Aufbringen der Elastifizierungsstreifen in gedehntem Zustand (S') auf die Hygieneartikel (D).

15. Verfahren nach einem der Ansprüche 12 bis 14, in Gang gesetzt mit der Vorrichtung nach einem der Ansprüche 1 bis 11.

16. Computerprogrammprodukt, das in den Speicher mindestens eines Computers (K, 106) ladbar ist und Softwarecodeteile enthält, die so konfiguriert sind, dass der Bewegungsvorgang der Ziehelemente (112) mit selektiv veränderlicher Geschwindigkeit (106, K) ausgelöst wird, wenn die Streifen verlegt werden, um den Aufbringabstand (P2) der Streifen in gedehntem Zustand (S') auf den Hygieneartikeln (D) in dem Verfahren nach einem der Ansprüche 12 bis 15 selektiv zu verändern, wenn das Produkt auf einem Computer abgearbeitet wird.

## Revendications

1. Dispositif destiné à former des bandes d'élastification pour application dans un état étiré (S') sur des articles sanitaires (D), comprenant au moins une paire d'éléments de traction combinés (112) afin de fonctionner en relation de saisie sur les extrémités (A) desdites bandes pour recevoir lesdites bandes disposées en forme de pont dans un état non-étiré (S) au niveau d'une extrémité d'entrée (I) du dispositif (100), où lesdits éléments de traction (112) sont à une première distance correspondant à la longueur desdites bandes (S) dans un état non-étiré (S) ; lesdits éléments de traction (112) étant mobiles (102, 104) suivant des trajectoires divergeant l'une par rapport à l'autre afin d'étirer lesdites bandes disposées en forme de pont entre eux vers une extrémité de sortie (0) du dispositif (100), où lesdits éléments de traction sont à une deuxième distance, plus grande que ladite première distance et correspondant à la longueur desdites bandes dans un état étiré de manière élastique (S), le dispositif comprenant une unité d'alimentation (300) destinée à délivrer lesdites bandes dans un état non-étiré (S) à ladite extrémité d'entrée (I) du dispositif (100) avec un pas d'alimentation donné (P1),
**caractérisé en ce que** lesdits éléments de traction (112) sont déplacés par des moyens de moteur (104) avec une vitesse variable de manière sélective (106, K) lors du transfert desdites bandes entre ladite extrémité d'entrée (I) et ladite extrémité de sortie (0) pour faire varier de manière sélective le pas d'application (P2) desdites bandes dans un état étiré (S') sur lesdits articles sanitaires (D), la variation de la vitesse desdits éléments de traction (112) entre ladite extrémité d'entrée (I) et ladite extrémité de sortie (0) déterminant le pas d'application (P2) desdites bandes (S') dans un état étiré sur lesdits articles sanitaires (D).

2. Dispositif selon la revendication 1, dans lequel lesdits éléments de traction (112) agissent sur les extrémités desdites bandes (A) dans une relation de saisie sans serrage ou pincement.

3. Dispositif selon l'une des revendications 1 ou 2, dans lequel :
- une unité d'alimentation (300) est prévue pour délivrer lesdites bandes (S) dans un état non-étiré à ladite extrémité d'entrée (I) du dispositif (100) à une vitesse correspondant à la vitesse d'application (V2) desdites bandes dans un état étiré (S') sur lesdits articles sanitaires (D), et
- chacun desdits éléments de traction (112) est inclus dans une paire d'éléments de traction opposés (112) donc, quand un élément de traction (112) de la paire est situé au niveau de ladite extrémité d'entrée (I), l'autre élément de traction (112) de la paire est situé au niveau de ladite extrémité de sortie (0).

4. Dispositif selon l'une quelconque des revendications précédentes, dans lequel lesdits éléments de traction (112) sont portés par des roues (102) disposées dans une position l'une en face de l'autre avec les axes de rotation respectifs (X102) incidents et obliques l'un par rapport à l'autre, l'inclinaison desdites axes (X102) déterminant le rapport entre ladite deuxième distance et ladite première distance et le degré d'étirement consécutif desdites bandes dans ledit état étiré (S').

5. Dispositif selon la revendication 3 et 4, dans lequel ladite paire d'éléments de traction opposés (112) comprend des éléments de traction (112) portés dans des positions diamétralement opposées par une desdites roues (102) disposées en position l'une en face de l'autre.

6. Dispositif selon la revendication 1 ou 3, dans lequel ladite unité d'alimentation (300) est un dispositif de coupe (302, 304) qui produit lesdites bandes (S) à partir d'une nappe de matière (200).

7. Dispositif selon l'une quelconque des revendications 4 ou 5, en combinaison avec la revendication 6, dans lequel ledit dispositif de coupe est un dispositif de coupe du type à coupe et glissement, approprié pour espacer lesdites bandes (S) l'une de l'autre quand celles-ci sont coupées à partir de ladite nappe (200).

8. Dispositif selon l'une quelconque des revendications précédentes dans lequel lesdits éléments de traction (112) comprennent, au moins un, et de préférence chacun des deux de :
- une unité de saisie à dépression (114), et
- des formations d'accrochage (120),
pour agir sur les extrémités desdites bandes (S, S').

9. Dispositif selon la revendication 8, dans lequel ladite unité de saisie à dépression (114), si elle est prévue, peut être reliée à une source de pression en dessous de l'atmosphère (118) par une conduite de raccordement pneumatique (116, 118) dans laquelle l'application de la pression en dessous de l'atmosphère sur ladite unité de saisie à dépression (114) peut être désactivée de manière sélective à ladite extrémité de sortie (0).

10. Dispositif selon l'une quelconque des revendications 8 ou 9, dans lequel lesdites formations d'accrochage (120) ont un profil général en dent de scie, de préférence avec le côté raide de la dent de scie orienté vers l'extérieur de la paire d'éléments de traction combinés (112).

11. Dispositif selon l'une quelconque des revendications 8 à 10, dans lequel lesdites formations d'accrochage sont disposées dans une position interposée entre lesdits éléments de saisie à dépression (114).

12. Procédé destiné à former des bandes d'élastification pour application dans un état étiré (S') sur des articles sanitaires (D) en utilisant au moins une paire d'éléments de traction combinés (112) fonctionnant en relation de saisie sur les extrémités (A) desdites bandes, le procédé comprenant le fait de :
- délivrer lesdites bandes dans un état non-étiré (S) aux dits éléments de traction (112) avec un pas d'alimentation donné (P1),
- recevoir lesdites bandes disposées en forme de pont dans un état non-étiré (S) entre lesdits éléments de traction (112) situés à une première distance correspondant à la longueur desdites bandes (S) dans l'état non-étiré (S) ;
- déplacer lesdits éléments de traction (112) suivant des trajectoires divergeant l'une de l'autre, lesdits éléments de traction étirant ainsi lesdites bandes disposées en forme de pont entre avec lesdits éléments de traction (112) situés à une deuxième distance, plus grande que ladite première distance et correspondant à la longueur desdites bandes dans un état étiré de manière élastique (S), **caractérisé en ce qu'**il comprend le fait de déplacer lesdits éléments de traction (112) avec une vitesse variable de manière sélective (106, K) lors du transfert desdites bandes pour faire varier de manière sélective le pas d'application (P2) desdites bandes dans un état étiré (S') sur lesdits articles sanitaires (D), la variation de la vitesse desdits éléments de traction (112) déterminant le pas d'application (P2) desdites bandes (S') dans un état étiré sur lesdits articles sanitaires (D).

13. Procédé selon la revendication 12, comprenant le fait de faire agir lesdits éléments de traction (112) sur les extrémités desdites bandes (A) dans une relation de saisie sans serrage ou pincement.

14. Procédé selon la revendication 12 ou 13, comprenant le fait d'appliquer lesdites bandes d'élastification dans un état étiré (S') sur lesdits articles sanitaires (D).

15. Procédé selon l'une quelconque des revendications 12 à 14, mis en oeuvre avec le dispositif selon l'une quelconque des revendications 1 à 11.

16. Produit de programme informatique pouvant être chargé dans la mémoire d'au moins un ordinateur (K, 106) et comprenant des parties de code de logiciel configurées pour mettre en oeuvre l'opération de déplacement desdits éléments de traction (112) avec une vitesse variable de manière sélective (106, K) lors du transfert desdites bandes afin de faire varier de manière sélective le pas d'application (P2) desdites bandes dans un état étiré (S') sur lesdits articles sanitaires (D) dans le procédé selon l'une quelconque des revendications 12 à 15 quand le produit est exécuté sur un ordinateur.
